# EUROPEAN PATENT SPECIFICATION

(11) **EP 2 708 211 B1**
(45) Date of publication and mention of the grant of the patent: **22.02.2017**
(21) Application number: 12184312.2
(22) Date of filing: 13.09.2012
(51) Int. Cl.: A61F 5/01, A61F 2/50, A61F 5/14

(54) **Method and apparatus for manufacturing orthotic device, and orthotic device**
Verfahren und Vorrichtung zum Herstellen einer Orthesevorrichtung und Orthesevorrichtung
Procédé et appareil de fabrication d'un dispositif orthétique et dispositif orthétique

(43) Date of publication of application: 19.03.2014
(73) Proprietor: Peacocks Orthotics Limited, Newcastle upon Tyne Tyne and Wear NE6 4NQ (GB)
(72) Inventor: PALLARI, Jari Heikki Petteri, Tyne and Wear, NE7 7QJ (GB)
(74) Representative: Vinsome, Rex Martin

(56) References cited:
- WO-A2-2011/031971
- DE-A1-102010 019 845
- US-A1- 2002 014 024

## Description

The present invention relates to a method for manufacturing an orthotic device and relates particularly, but not exclusively, to a method for manufacturing foot orthoses.

Foot orthoses, also known as orthopaedic insoles, are corrective medical devices which are placed inside a user's shoe in order to realign the musculoskeletal system of the user while the user is walking, or to redistribute pressure on the plantar surface of the user's foot. Known foot orthoses generally consist of form fitting insoles which may be provided with additional wedges, pads, bars, cut-outs and other features and/or different combinations of materials, or hard and/or soft covering materials. Foot orthoses are usually worn in pairs and are used to treat a variety of foot and lower limb pathologies. They may be also used to treat musculoskeletal conditions in the whole body that may be influenced by altering the forces in the foot and to treat pathologies that may manifest themselves in the foot that arise from medical conditions such as diabetes etc. Foot orthoses are also used for injury prevention in sports.

A known orthotic device is disclosed in EP2196173. This discloses an insole manufactured by means of an additive manufacturing process and having a heel structure that can have different properties depending on the structure integrated into the heel. However, this orthotic device suffers from the drawback that the mechanical properties, for example compressibility, of the orthotic are determined by those properties of the material formed from the additive manufacturing process, which in turn limits the compressibility and fatigue performance of the orthotic device as a whole.

DE 102010019845 discloses a method according to the preamble of claim 1.

A further known method is disclosed in US 2002/0014024.

Preferred embodiments of the present invention seek to overcome one or more of the above disadvantages of the prior art.

According to the present invention, there is provided a method of manufacturing according to claim 1.

By forming a body defining at least one recess for at least partially enclosing at least one insert, by means of an additive manufacturing process using data representing dimension of a body part of a user, this provides the advantage of enabling a wider range of possible mechanical properties of the orthotic device to be selected, thereby enabling a wider range of treatments to be carried out using the orthotic device.

The data may comprise a 3D representation of a body part of the user.

The body may define a plurality of said recesses.

This provides the advantage of increasing the range of properties of the device, thereby increasing the range of treatments that can be carried out.

A plurality of said recesses may be separated in a direction perpendicular to a weight bearing direction of the device.

A plurality of said recesses may be separated in a direction parallel to a weight bearing direction of the device.

The method may further comprise forming at least one aperture in a wall of at least one said recess.

This provides the advantage of enabling the range of flexibilities of the orthotic device to be increased.

A preferred embodiment of the method according to the present invention will now be described, by way of example only and not in any limitative sense, with reference to the accompanying drawings. The apparatus for manufacturing and the orthoric device described in relation to the figures do not form part of the scope of the claims of the present invention.
Figure 1 is a schematic view of an apparatus usefull in connection with carrying out the method for manufacturing an orthotic device according to the present invention;
Figure 2 is a perspective view from the front and above of an orthotic device manufactured by means of the apparatus of Figure 1;
Figure 3 is a perspective view from the front and below of the orthotic device of Figure 2;
Figure 4 is a cross sectional view through a heel portion of the orthotic device of Figure 2;
Figure 5 is a view corresponding to Figure 3 of an orthotic device obtained by means of the method of the present invention;
Figure 6 is a view corresponding to Figure 4 of the orthotic device of Figure 5;
Figures 7 to 10 show cross sectional views through heel sections of orthotic devices obtained by means of the method of the present invention;
Figures 11 and 12 show plan views of further orthotic devices obtained by means of the method of the present invention;
Figure 13 is a side view of an orthotic device obtained by means of the method of the present invention;
Figure 14 is a schematic cross sectional view of an enlarged part of Figure 13; and
Figures 15 and 16 are plan views of further orthotic devices obtained by means of the method of the present invention.

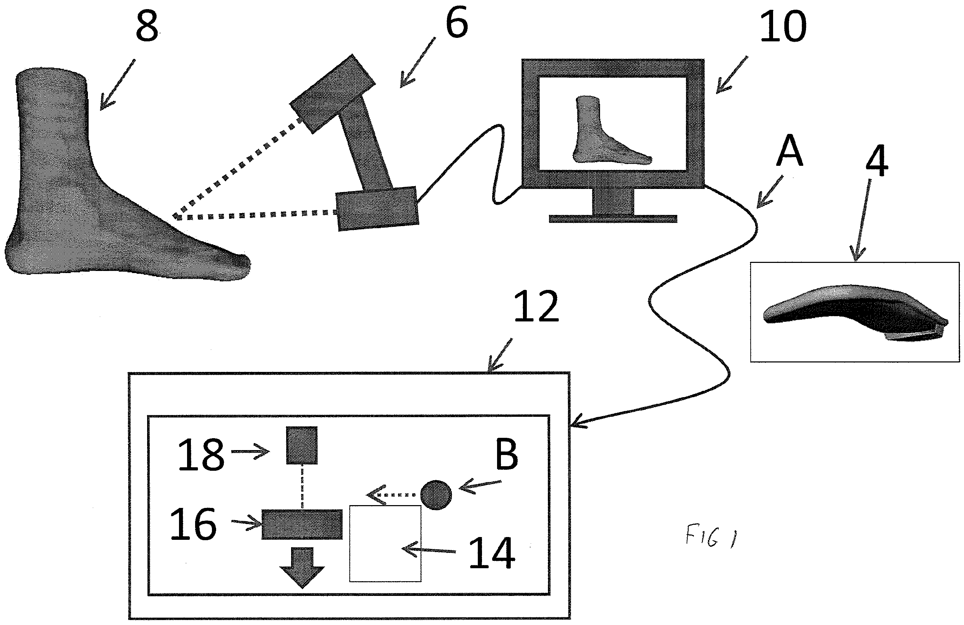

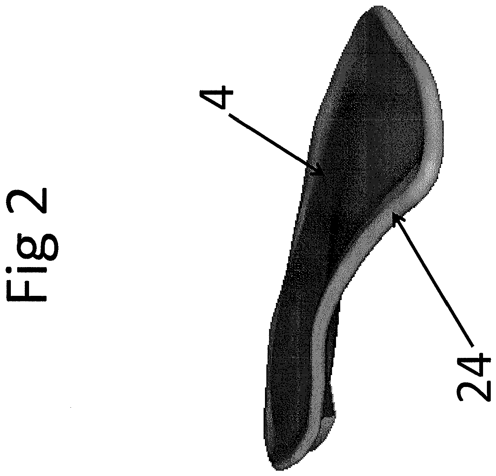

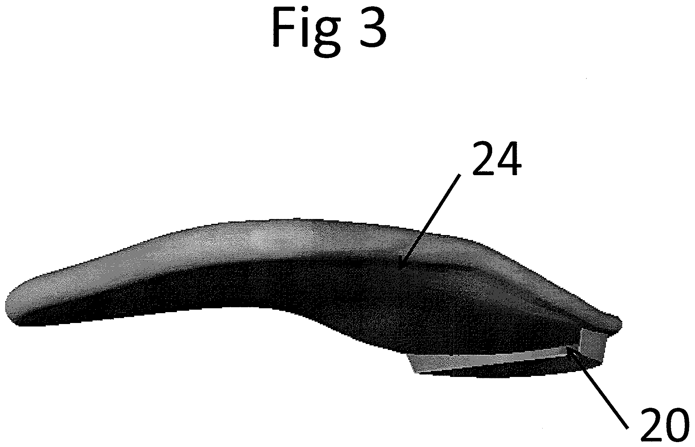

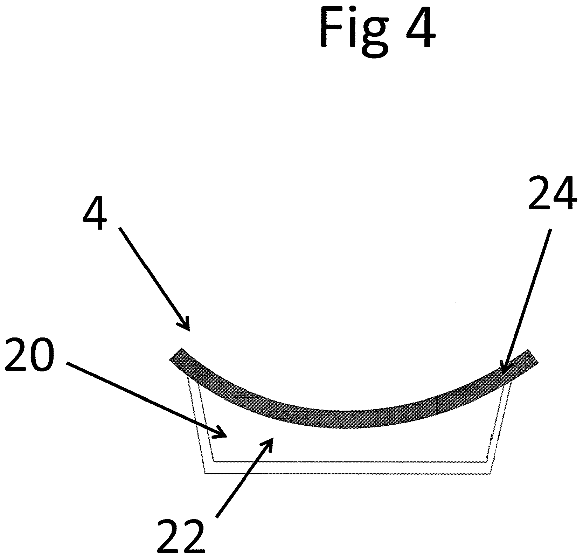

Referring to Figure 1, an apparatus 2 for manufacturing an orthotic device 4 (Figure 2) such as a foot orthosis includes an optical scanner 6 for obtaining data relating to dimensions of a user's foot 8, and a computer 10 for receiving and processing the data to provide a 3D representation of the users foot 8, for designing a digital representation of the orthotic device 4 and digitally sending it via connection 11 to an apparatus 12 for depositing plastic powder 14 onto a moving platform 16 by means of a roller 17 under the control of the computer 10. For example, the apparatus 12 can be a commercially available selective laser sintering machine, for example as provided under the trade mark sPro 60 by 3D Systems Inc. A laser device 18 selectively melts the plastic powder 14 to produce successive layers of a body 24 (Figure 2) of the orthotic device 4 such that the body defines one or more recesses 20 (Figure 3) for receiving a filler 22 (Figure 4) of elastomeric material such as a silicone polymer. It will be appreciated by persons skilled in the art that instead of a direct scan being made of the user's foot, the foot shape can also be captured by scanning a foam impression of the foot or by scanning a "slipper cast" of the foot. In addition, other additive manufacturing techniques familiar to persons skilled in the art can be used, such as a fused deposition modelling process, stereolithography, digital light processing, fused filament fabrication, laminated object manufacturing, the polyjet process or processes using powder bed and inkjet head 3D printing.

Referring to Figures 2 to 4, the orthotic device 4 produced using the apparatus of Figure 1 has a body 24 defining one or more recesses 20 for receiving a filler 22 elastomeric material for influencing the mechanical properties of the orthotic device 4 as a whole.

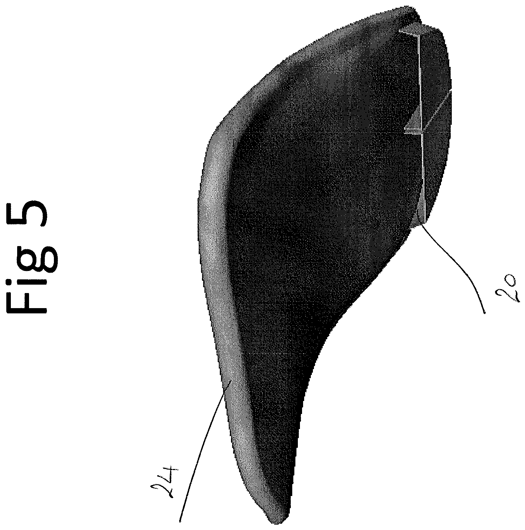

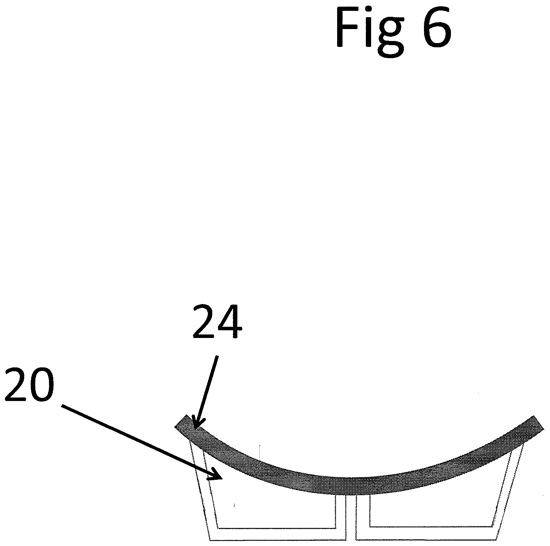

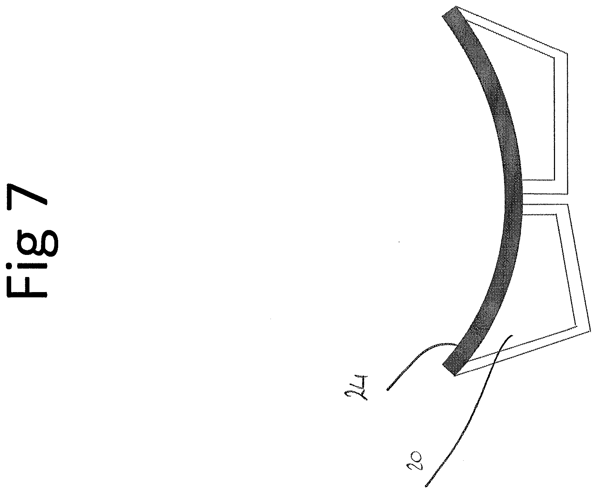

In a further example, as shown in Figures 5 and 6, more than one cavity 20 can be built under the heel of the orthotic device. If the properties between the medial and lateral side of the heel are different (soft-hard), the body 24 will be forced to move differently as one side will "give in" more easily and the other will resist the deformation more. This will enable further options for controlling the movement of the person wearing the orthotic device 4. The cavities 20 can also be in the form of a medial/lateral wedge as shown in Figure 7.

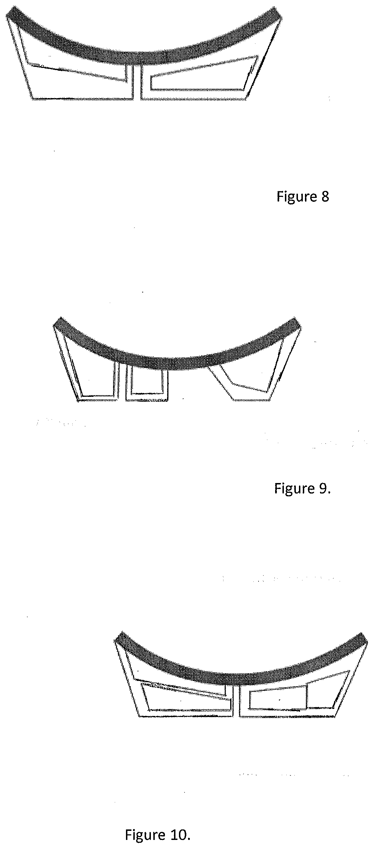

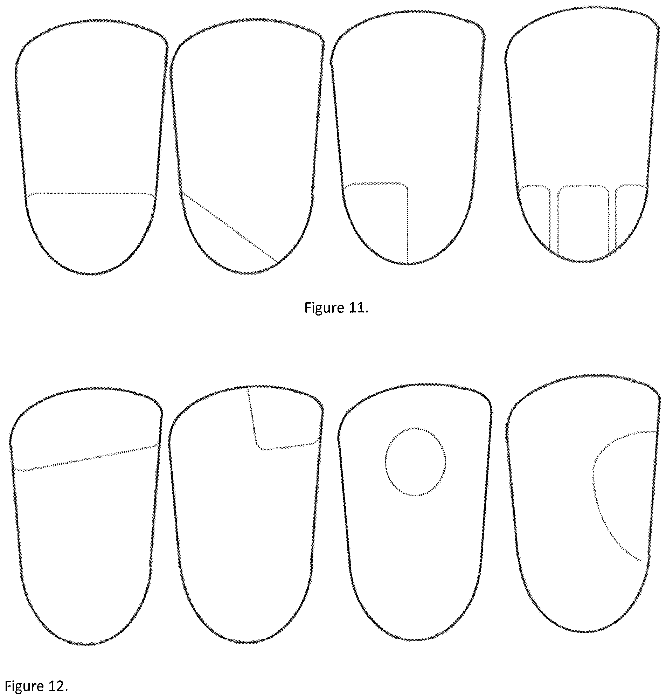

Further examples are shown using more complex combinations with three or more cavities 20 to provide shock absorption directly under the heel and dynamic control with different compliance in different parts of the heel structure, as shown in Figures 7 to 10. As shown in Figure 11, the heel and cavity shapes can also be altered in many ways to provide different responses to loading.

In further examples, cavities 20 can be built into other parts of the orthotic shell 4, such as under the arch or the forefoot, on top of the shell or below the shell, or on top of the shell to form further supporting or pressure relieving features, such as metatarsal pads/bars as presented in Figure 12.

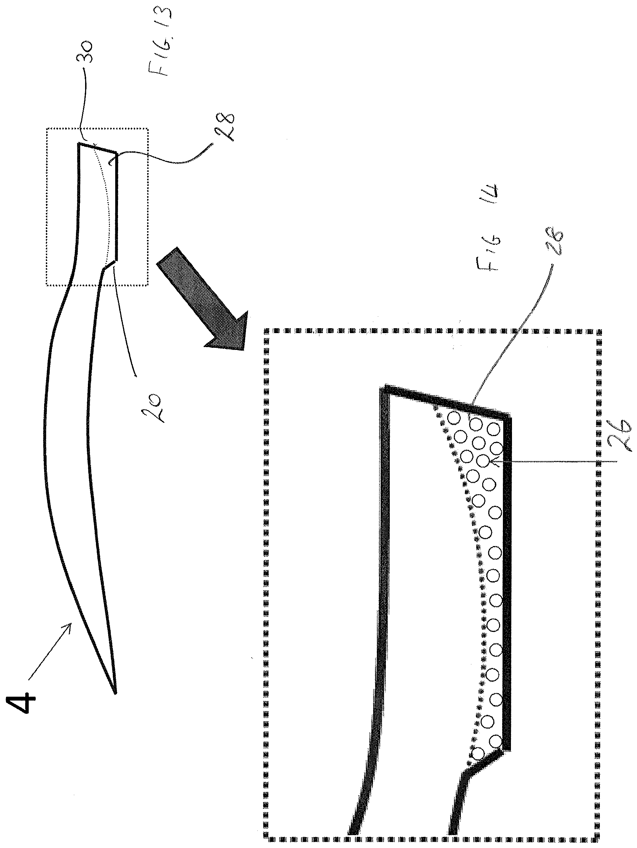

Referring to Figures 13 and 14, a further examples of the orthotic device 4 has a series of holes 26 provided in a wall 28 of the recess 20. The holes 26 generally extend through the wall of the recess 20 but not through the external wall 30 of the shell of the orthotic device 4. The holes 26 extend generally perpendicularly to the weight bearing direction of the device. During the process of injecting the insert material of filler 22 (Figure 4), the holes 26 can be covered with tape or similar material, which can then be removed once the filler 22 has set.

The purpose of the holes 26 is to modify the stiffness of the recess walls, since a wall with holes 26 will be more flexible than a wall without holes. If the recess wall does not provide resistance to the deformation of the orthotic device 4 while weight is being applied to it, the inserted polymer 22 will have to carry the load. This is desirable as the fatigue properties of the insert 22 can be better than the material of the body 24 of the orthotic device 4. In addition, the holes 26 can provide a pleasing appearance to the orthotic device 4.

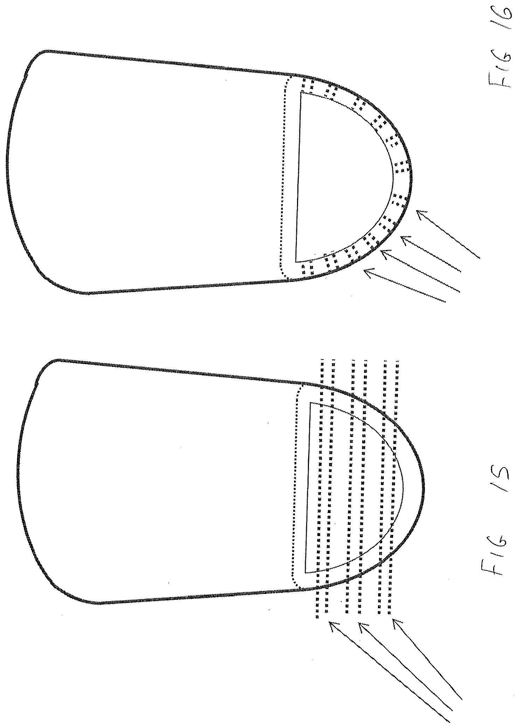

With reference to Figures 15 and 16, the holes 26 can be created in the CAD environment during manufacture of the body 24 of the orthotic device 4 by projecting the hole shape through the wall of the relevant recess 20. The projection can be generally perpendicular to the longitudinal axis of the orthotic device 4 (Figure 15) or generally perpendicular to the normal of the recess wall surface (Figure 16). The cross-section of the holes 26 can be circular, rectangular, elliptical, triangular, hexagonal or any other geometry that can be defined by a mathematical equation or an artist's sketch. The holes 26 can also be arranged in a regular pattern or arranged freely in the space available.

All of the features described previously can be combined together and the fillers for the different cavities can be also different. For example a two-part heel design with materials A and B in the medial and lateral compartments can be combined with a metatarsal pad filled with material C.

The present invention provides significant advantages over known customised foot orthotics with customised silicone components that are currently made either manually with plaster and thermoplastics or via a milling based process from foams, such as EVA or from blocks of plastic, such as polypropylene. In particular, the known processes are time-consuming and the technician has limited control over the design of the device and how it works. If the features are placed manually, they are subject to the skill and experience of the technician carrying out assembly. This gives rise to inconsistency in the form of lack of repeatability with the products. If a CAD system is involved in the design, only the body of the orthotic can be designed and any external components will have to be added manually after the milling. With the present invention, all of the above takes place in a digital environment allowing the designer not only to control numerically all aspects of the geometry of the product but also to utilise a number of analytical engineering tools - such as finite element optimisation - and to use different digital measurement techniques such as pressure measurements, magnetic resonance or tomographic imaging methods directly in the design environment. The features designed/placed using these techniques can ensure that the orthotic device works better, fits better and is more comfortable.

It will be appreciated by persons skilled in the art that the above embodiments have been described by way of example only and not in any limitative sense, and that various alterations and modifications are possible without departure from the scope of the invention as defined by the appended claims.

**Reference number table**

| | |
|---|---|
| 2 | Apparatus for manufacturing orthotic device |
| 4 | Orthotic device |
| 6 | Optical scanner |
| 8 | Foot |
| 10 | Computer |
| 12 | Apparatus for depositing plastic powder |
| 14 | Plastic powder |
| 16 | Moving platform |
| 17 | Roller |
| 18 | Laser device |
| 20 | Recess |
| 22 | Filler |
| 24 | Body |
| 26 | Holes |
| 28 | Wall |
| 30 | External wall |

## Claims

1. A method of manufacturing an orthotic device, the method comprising:-
inputting, via input means (10), data representing dimensions of a body part (8) of a user; and
forming, via forming means (12), by means of an additive manufacturing process using said data, a body (24) of an orthotic device (4), the body defining at least one recess (20) for at least partly enclosing at least one respective insert (22);
**characterised by** inserting at least one respective elastomeric insert into at least one said recess by means of injection of mouldable material.

2. A method according to claim 1, including one or more of the following features:
(i) wherein the data comprises a 3D representation of a body part of the user;
(ii) wherein the body defines a plurality of said recesses; or
(iii) further comprising forming at least one aperture (26) in a wall (28) of at least one said recess.

3. A method according to claim 2, including one or more of the following features:
(i) wherein a plurality of said recesses are separated in a direction perpendicular to a weight bearing direction of the device; or
(ii) wherein a plurality of said recesses are separated in a direction parallel to a weight bearing direction of the device.

## Patentansprüche

1. Verfahren zur Herstellung einer Orthesevorrichtung umfassend:
Eingabe von Daten, die Maße eines Körperteils (8) eines Anwenders darstellen, mit Hilfe eines Eingabemittels (10); und
Formen eines Körpers (24) einer Orthesevorrichtung (4) mit Hilfe eines Formungsmittels (12), mittels eines additiven Herstellungsverfahrens, das die Daten verwendet, wobei der Körper mindestens eine Aussparung (20) zur mindestens teilweisen Umschließung mindestens einer entsprechenden Einlage (22) ausbildet;
**dadurch gekennzeichnet, dass** mindestens eine elastomere Einlage in die mindestens eine Aussparung mittels Einspritzung eines formbaren Materials eingesetzt wird.

2. Verfahren nach Anspruch 1, enthaltend eines oder mehrere der folgenden Merkmale:
i. wobei die Daten eine 3D-Darstellung eines Körperteils des Anwenders umfassen;
ii. wobei der Körper eine Vielzahl der Aussparungen ausbildet; oder
iii. ferner umfassend Bilden mindestens einer Öffnung (26) in einer Wand (28) mindestens einer der Aussparungen.

3. Verfahren nach Anspruch 2, enthaltend eines oder mehrere der folgenden Merkmale:
i. wobei eine Vielzahl der Aussparungen in einer Richtung rechtwinklig zu einer Belastungsrichtung der Vorrichtung unterteilt sind; oder
ii. wobei eine Vielzahl der Aussparungen in einer Richtung parallel zu einer Belastungsrichtung der Vorrichtung unterteilt sind.

## Revendications

1. Procédé de fabrication d'un appareillage orthopédique, le procédé comprenant :
l'entrée, via un moyen d'entrée (10), de données représentant des dimensions d'une partie corporelle (8) d'un utilisateur ; et
la formation, via un moyen de formation (12), au moyen d'un processus de fabrication additive à l'aide desdites données, d'un corps (24) d'un appareillage orthopédique (4), le corps définissant au moins un évidement (20) destiné à au moins en partie enserrer au moins un insert (22) respectif ;
**caractérisé par** l'insertion d'au moins un insert élastomérique respectif dans au moins un dit évidement au moyen de l'injection d'un matériau moulable.

2. Procédé selon la revendication 1, incluant une ou plusieurs des particularités suivantes :
(i) dans lequel les données comprennent une représentation en 3D d'une partie corporelle de l'utilisateur ;
(ii) dans lequel le corps définit une pluralité desdits évidements ; ou
(iii) comprenant en outre la formation d'au moins une ouverture (26) dans une paroi (28) d'au moins un dit évidement.

3. Procédé selon la revendication 2, incluant une ou plusieurs des particularités suivantes :
(i) dans lequel une pluralité desdits évidements sont séparés dans une direction perpendiculaire à une direction porteuse de poids de l'appareillage ; ou
(ii) dans lequel une pluralité desdits évidements sont séparés dans une direction parallèle à une direction porteuse de poids de l'appareillage.
